# EUROPÄISCHE PATENTSCHRIFT

(11) **EP 2 117 474 B1**
(45) Veröffentlichungstag und Bekanntmachung des Hinweises auf die Patenterteilung: **27.04.2011**
(21) Anmeldenummer: 08707466.2
(22) Anmeldetag: 31.01.2008
(51) Int. Cl.: A61F 2/58

(54) **PROTHESENFINGER**
PROSTHETIC FINGER
DOIGT PROTHÉTIQUE

(30) Priorität: 01.02.2007 DE 102007005858
(43) Veröffentlichungstag der Anmeldung: 18.11.2009
(73) Patentinhaber: Otto Bock Healthcare Products GmbH, 1070 Wien (AT)
(72) Erfinder: PUCHHAMMER, Gregor, A-1130 Wien (AT); HASLINGER, Martin, A-3653 Weiten (AT)
(74) Vertreter: Stornebel, Kai
(86) Internationale Anmeldenummer: PCT/EP2008/000779
(87) Internationale Veröffentlichungsnummer: WO 2008/092695

(56) Entgegenhaltungen:
- EP-A- 1 195 151
- WO-A-2005/025462
- DE-A1- 19 854 762

## Beschreibung

Die Erfindung betrifft einen Prothesenfinger mit einem Proximalglied, einem Medialglied und einem Distalglied, die verschenkbar ineinander gelagert sind und einem in dem Prothesenfinger angeordneten Motor, der über eine Getriebeeinrichtung das Medialglied relativ zu dem Proximalglied verdreht. Ein solcher Prothesenfinger ist insbesondere für künstliche Hände geeignet.

An die moderne Prothetik werden aufgrund der fortschreitenden Möglichkeiten zur neuronalen Kopplung entsprechender Antriebe immer größere Anforderungen an eine separate Aktuierbarkeit einzelner Komponenten gestellt. Neben den üblichen Forderungen nach einer möglichst leichten Prothese, die zuverlässig ist und möglichst preiswert ist, wird hinsichtlich der Funktionsweise eine möglichst große Annäherung an die des zu ersetzenden Körpergliedes gewünscht. Die Prothese soll möglichst sämtliche Tätigkeiten erlauben, die das ersetzte Körperglied ausüben kann. Dazu sind in der Regel eine Vielzahl von Antrieben und Steuerungseinrichtungen sowie eine erhöhte Anzahl von Gelenkverbindungen notwendig.

Insbesondere bei Handprothesen werden aufgrund des filigranen Aufbaus der Hand und der außerordentlich hohen Flexibilität große Anforderungen an eine entsprechende Prothese gestellt.

Um beispielsweise Prothesenfinger relativ zu einem Chassis zu verlagern, sind im Chassis oder in einer Unterarmprothese Antriebe vorgesehen, die über Zugmittel die Prothesenfinger palmar und dorsal verlagern. Die Zugmittel übernehmen dadurch die Aufgaben der menschlichen Sehnen.

Um eine präzisere Steuerung der Prothesenfingerbewegung zu ermöglichen, wobei die Finger in ihrer Größe und Abmessung so kompakt sind, dass sie den menschlichen Fingern möglichst genau entsprechen, schlägt die DE 198 54 762 A1 vor, dass im proximalen und medialen Fingerglied ein Motor angeordnet ist, der über ein Getriebe das jeweils nachfolgende Prothesenfingerglied verschwenkt. Die Ansteuerung erfolgt über neuronale Signale. In dem Medialglied ist ein Motor angeordnet, der das Distalglied verschwenkt. Ein Verlängerungsteil, das mit einer künstlichen Fingerkuppe verbunden werden kann, ist über ein Zahnradgetriebe mit Kopplungszahnrädern mit einem feststehenden Zahnrad gekoppelt. Wird der Motor des medialen Fingergliedes geschaltet, bewegt sich das distale Fingerglied nach oben oder unten. Entsprechend der Bewegung der Kopplungszahnräder kippt das Verlängerungsteil nach unten oder oben. Die Getriebeanordnung des distalen Fingergliedes gleicht den relativ langen Hebel, der durch das Verbindungsglied und das Verlängerungsteil gebildet wird, aus. Wird mit der Fingerkuppe ein Gegenstand gegriffen und festgehalten, bleibt das distale Fingerglied auch nach Abschalten des Motors des medialen Fingergliedes aufgrund seiner Schneckenradkopplung in der entsprechenden Stellung fest und starr.

Diese Anordnung benötigt eine Vielzahl von Teilen, insbesondere eine Vielzahl von Motoren und Getrieberädem, so dass die Konstruktion insgesamt aufwendig ist.

Aufgabe der vorliegenden Erfindung ist es, einen Prothesenfinger bereitzustellen, der leichter ausgestaltet ist, ohne dass die Funktionsfähigkeit nachteilig beeinflusst wird.
Erfindungsgemäß wird diese Aufgabe durch einen Prothesenfinger mit den Merkmalen des Anspruchs 1 gelöst. Vorteilhafte Ausgestaltungen und Weiterbildungen der Erfindung sind in den Unteransprüchen aufgeführt.

Der erfindungsgemäße Prothesenfinger mit einem Proximalglied, einem Medialglied und einem Distalglied, die verschwenkbar ineinander gelagert sind, und einem in dem Prothesenfinger angeordneten Motor, der über eine Getriebeeinheit das Medialglied relativ zu dem Proximalglied verdreht, sieht vor, dass in dem Medialglied ein längsbeweglicher Waagebalken angeordnet ist, der über Hebel mit dem Proximalglied und dem Distalglied verbunden ist. Durch die längsverschiebliche Ausgestaltung des Waagebalkens, der über Verstellhebel mit sowohl dem Proximalglied als auch mit dem Distalglied verbunden ist, wird gewährleistet, dass mit nur einem Antrieb zwei Fingerglieder gleichmäßig bewegt werden, so dass sich der Finger harmonisch abwinkelt. Dabei werden zwei Gelenke nur von einer Antriebseinheit bewegt, so dass trotz des Vorliegens eines unteraktuierten Systems mit zwei Freiheitsgraden und einem Bewegungsgrad eine vollständige Beweglichkeit des Prothesenfingers gegeben ist. Der Motor ist aus Gründen der Gewichtsverteilung bevorzugt in dem Proximalglied gelagert, kann aber auch in dem Medialglied untergebracht sein. Prinzipiell ist eine Anordnung im Distalglied ebenfalls möglich, dies würde aber den Schwerpunkt sehr weit von der Lagerung des Prothesenfingers weg verlagern und aufgrund der beengten Platzverhältnisse nur die Montage eines kleinen Motors und damit eine geringe Griffkraft ermöglichen.

Der Waagebalken ist bevorzugt an einer Spindelmutter gelagert oder angeordnet, die sich bei Drehung der angetriebenen Spindel entlang der Längserstreckung des Medialgliedes bewegt. Die Bewegungsrichtung ist dabei von der Drehrichtung der Spindel abhängig. Die Drehbewegung der Spindel um eine Achse parallel zur Längserstreckung des Medialgliedes bewirkt, dass die Spindelmutter und damit der daran festgelegt Waagebalken leicht translatorisch bewegt werden kann. Der Aufbau der Waagebalkenkinematik ermöglicht einen kompakten Aufbau, wodurch die einzelnen Komponenten in ihren geometrischen Abmessungen innerhalb der Kontur eines normalen Medialgliedes untergebracht werden können.

Der Motor ist mit der Spindel gekoppelt, die drehbeweglich in dem Medialglied gelagert ist, vorzugsweise ist die Spindel auf einer Zentralachse des Medialgliedes gelagert, so dass sich ein symmetrischer Aufbau der Fingerprothese erreichen lässt. Eine außermittige Anordnung der Spindel kann ebenfalls vorgesehen sein.

Zur Verringerung der Reibungsverluste ist die Spindel als eine Kugelumlaufspindel ausgebildet, so dass sehr hohe Wirkungsgrade aufgrund der geringen Reibung zwischen der Spindel und der Spindelmutter erzielt werden können. Dadurch ist für den Antrieb nur ein entsprechend klein dimensionierter Motor notwendig, so dass der Antrieb und die Spindel mit Spindelmutter sehr leicht dimensioniert werden können. Aufgrund der nicht vorhandenen Selbsthemmung der Kugelumlaufspindel sind weitere Maßnahmen zu treffen, damit bei einer abtriebsseitigen Belastung der Motor nicht beschädigt wird.

Um eine symmetrische Kraftverteilung innerhalb des Prothesenfingers zu gewährleisten, sind die Hebel, die die Spindelmutter mit dem Proximalgelenk und dem Distalgelenk verbinden, bevorzugt beidseitig an der Spindelmutter angeordnet, so dass eine Verkantung der Spindelmutter nicht stattfinden kann.

Bevorzugt sind die Spindel und der Motor über ein Kegelradgetriebe miteinander gekoppelt, wobei bei einer Anordnung des Motors im Proximalglied ein Kopplungskegelrad um die Drehachse des Medialgelenkes am Proximalglied gelagert ist. Auf diese Weise ist es möglich, über ein an der Abtriebswelle des Motors angeordnetes Kegelrad die Spindel, die ebenfalls ein Kegelrad aufweist, drehwinkelunabhängig anzutreiben.

Analog zu der Lösung der beidseitig angeordneten Hebel ist es vorgesehen, dass zwei Waagebalken beiderseits der Spindelmutter angeordnet sind, um eine gleichmäßige Kraftverteilung zu bewirken. Um die Bewegung der distalen und proximalen Hebel zu synchronisieren, sind die Waagebalken miteinander verbunden, so dass die beiden Waagebalken eine Brücke oder einen Rahmen um die Spindelmutter herum bilden.

Die Waagebalken können entweder in einem festgelegten Winkel zu der Bewegungsrichtung festgelegt sein, bei einer Anordnung auf einer Spindelmutter drehfest an der Spindelmutter befestigt, oder um einen festgelegten Winkel schwenkbar zur Bewegungsrichtung ausgebildet sein, um Ungleichmäßigkeiten in der Beugebewegung des Medial- und Distalgelenkes ausgleichen zu können. Bei einer beweglichen Lagerung des Waagebalkens, beispielsweise an der Spindelmutter, ist vorteilhafterweise vorgesehen, dass eine Normalposition unter Federvorspannung gehalten wird. Dazu ist der Waagebalken elastisch gegenüber der Spindelmutter abgestützt. Grundsätzlich ist es auch möglich, dass in beiden Drehrichtungen, ausgehend von der Normalposition, eine Verschwenkung zugelassen wird und elastische Elemente, beispielsweise Federn oder Kunststoff- bzw. Gummiteile, den Waagebalken vorgespannt in der Normalposition halten. Wird nun der Finger mit Leerlast bewegt, also bevor sich das Medial- und Distalgelenk um einen Gegenstand herum legen, wird der Waagebalken in dem Winkel gehalten. Bei einer Objektberührung des Medial- oder Distalgliedes, wenn das jeweils andere Prothesenfingerglied nicht von einem Objekt aufgehalten wird, bewegt sich das jeweils nicht aufgehaltene Prothesenfingerglied weiter, da die Spindelmutter weiter verlagert wird. Durch die Zulassung einer Veränderung des Positionswinkels des Waagebalkens relativ zur Bewegungsrichtung kann sich das jeweils noch nicht im Kontakt befindliche Prothesenfingerglied eingeschränkt weiterbewegen. Dadurch kann eine ungleichmäßige Abwinkelung des Medialgliedes und Distalgliedes erfolgen, so dass die Kopplung über das Waagebalken-Hebelsystem als ein Differential funktioniert. Die Federvorspannung kann durch ein Federelement generiert werden, das eine lineare oder bevorzugt progressive Kennlinie aufweist, so dass ein entsprechendes Verhältnis zwischen dem Verdrehwinkel des Waagebalkens und dem Rückstellmoment erreicht wird.

Eine Weiterbildung der Erfindung sieht vor, dass die Spindel axial verlagerbar gelagert und bevorzugt am distalen Ende mit einer Sperreinrichtung versehen ist, die bei Überschreiten eines festgelegten Verschiebeweges mit der Spindel in Eingriff tritt und diese sperrt. Die Sperreinrichtung kann dabei als Formschluss- oder Reibschlussbremse ausgebildet sein, die eine weitere Drehbewegung der Kugelumlaufspindel bei Einleitung eines abtriebsseitigen Momentes über das Distalglied verhindert. Eine Ausgestaltung dieser Sperreinrichtung sieht vor, dass diese als eine elastische Aufnahme mit Reibflächen oder Formschlusselementen ausgebildet ist. Die Spindel kann in der Sperreinrichtung gelagert sein, so dass die Sperreinrichtung gleichzeitig als Lagerstelle dient. Wird ein abtriebsseitiges Moment eingeleitet, verlagert sich die Spindel distal und tritt dann in Eingriff mit den Reibschluss- oder Formschlusselementen und sperrt die Weiterbewegung. Innerhalb der Sperreinrichtung kann die Spindel mit einer Kugellagerung versehen sein, so dass im Normalbetrieb ein möglichst reibungsarmer Antrieb erfolgen kann.

Um die Spindel längsverschieblich lagern zu können, wenn entsprechende Belastungsszenarien auftreten, ist diese am Kegelrad längsverschieblich und drehfest gelagert, so dass das am Kopplungskegelrad gelagerte, spindelseitige Kegelrad stets in Eingriff bleibt. Die längsverschieblich und drehfeste Lagerung kann über eine polygonale Verzahnung erfolgen.

Eine Weiterbildung der Erfindung sieht vor, dass Sensoreinrichtungen an jedem Gelenk zur Erfassung der Winkelstellungen des jeweiligen Prothesefingergliedes vorhanden sind. Ebenfalls können Sensoreinrichtungen zur Erfassung der Belastung der jeweiligen Bauteile, insbesondere der Drehmomente oder Biegemomente mittels Dehnmessstreifen vorgesehen sein. Im Distalglied ist ein Sensor distal und/oder palmar orientiert angeordnet, um einen Kontakt der Prothesenfinger mit einem Objekt zu detektieren und gegebenenfalls dem Prothesennutzer eine Rückmeldung geben zu können.

Eine Steuerelektronik des Motors und eine Auswerteeinheit ist ebenfalls innerhalb des Prothesenfingers angeordnet, so dass eine prothesenfingerautarke Steuerung und Auswertung der Sensordaten erfolgen kann. Der Prothesenfinger kann somit als eine Baueinheit konstruiert und gefertigt werden, die nur noch mechanisch an einem Chassis angeschlossen und mit einer Busverbindung zur Weiterleitung der Steuerdaten und Sensordaten sowie mit einer Energieversorgung verbunden werden muss. Das Proximalglied kann ebenfalls angetrieben an dem Chassis gelagert sein. Die Steuerelektronik ist bevorzugt im Prothesenfinger, z.B. im Proximalglied, insbesondere unterhalb des Motors, angeordnet. Um die Steuereinheit mit den Sensoren zu verbinden, ist eine flexible Leiterbahn vorgesehen, die die Elektronikeinheit mit den Sensoren verbindet.

Der Motor kann mit einem Untersetzungsgetriebe gekoppelt sein, das bevorzugt als ein Reibradgetriebe ausgebildet ist. Dabei sind Motor und Getriebe als Baueinheit ausgebildet, um eine möglichst geringe Baugröße realisieren zu können. Der Abtrieb des Motors erfolgt über eine Motorwelle, die am Proximalende an einem Motorgehäuse und am Distalende innerhalb des Reibradgetriebes gelagert ist. Dadurch wird eine zweite Lagerstelle innerhalb des Motors eingespart, was die Länge der Motor-Getriebeeinheit insgesamt verringert.

Dem Motor, gegebenenfalls der Motor-Getriebeeinheit, ist ein Gesperre nachgeschaltet, das eine abtriebsseitige Lasteinleitung sperrt und nur antriebsseitige Momente weiterleitet. Dadurch kann bei Ausfall der Energieversorgung oder bei Abschaltung des Motors die eingenommene Stellung der Finger gehalten werden. Eine Selbsthemmung des Antriebes und damit eine Erhöhung der Verlustleistung ist dadurch nicht mehr notwendig. Das Gesperre ist dabei vorzugsweise als in beide Drehrichtungen wirkende Klemmrollenfreiläufe ausgebildet, die über eine Betätigung des Motors gelöst wird. Das Gesperre kann ebenfalls in der Motor-Getriebeeinheit integriert sein, wobei eine Lagerung des Gesperres durch Kugeln an einem Rollenhalter des Klemmrollenfreilaufs des Gesperres vorgesehen ist. Der Motor, das Getriebe und das Gesperre sind als Baueinheit ausgeführt und können auch zum Antrieb anderer Einrichtungen eingesetzt werden. Ein Reibradgetriebe hat den Vorteil eines geräuscharmen Laufes.

Gleiche Bezugszeichen in den Figuren bezeichnen gleiche Bauteile. Es zeigen:
- Figur 1-: eine Teilschnittansicht eines Prothesenfingers;
- Figur 2 -: eine Schnittdarstellung eines Prothesenfingers in Längsebene;
- Figur 3 -: eine Detaildarstellung eines Waagebalkengetriebes in gestreckter Stellung;
- Figur 4 -: eine Darstellung der Figur 3 in leicht gebeugter Stellung;
- Figur 5 -: eine Darstellung des Prothesenfingers in maximal gebeugter Stellung;
- Figur 6 -: eine Darstellung der Motoreinheit;

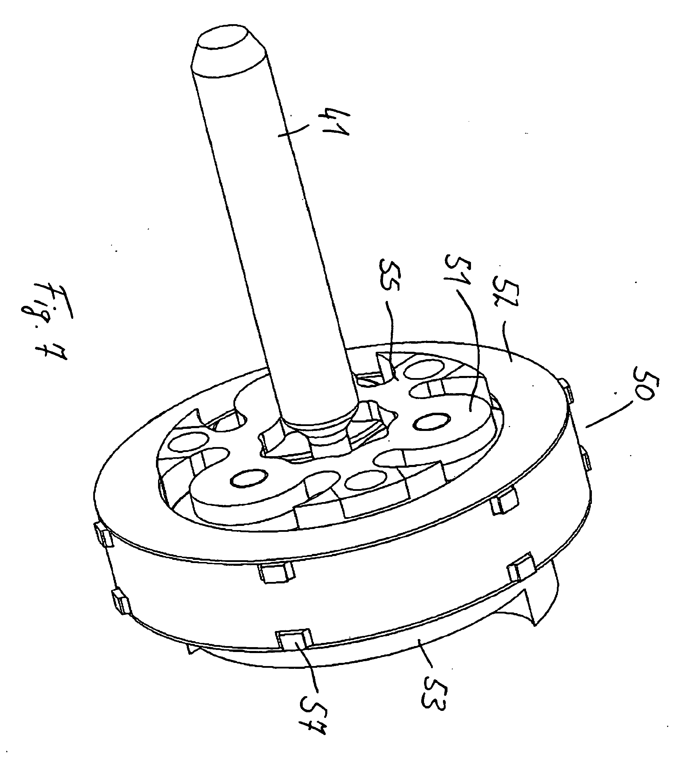

- Figur 7 -: eine Detaildarstellung eines Reibradgetriebes;
- Figur 8 -: eine Schnittdarstellung des Reibradgetriebes gemäß Figur 7;
- Figur 9 -: eine Detaildarstellung des Reibradgetriebes;
- Figur 10 -: eine abtriebsseitige Ansicht des Reibradgetriebes;
- Figur 11 -: eine Detaildarstellung eines Gesperres;
- Figur 12-: eine antriebsseitige Draufsicht auf das Gesperre;
- Figur 13 -: eine perspektivische Darstellung des Gesperres von Abtriebsseite;
- Figur 14 -: eine Schnittdarstellung durch die Motor-Getriebe- Gesperreeinheit;
- Figur 15 -: eine Detaildarstellung eines Kegelradgetriebes;
- Figur 16 -: eine perspektivische Darstellung der Figur 15;
- Figur 17 -: eine Detaildarstellung einer Spindel mit Spindelmutter und Waagebalken;
- Figur 18 -: eine Spindelmutter in Einzeldarstellung;
- Figur 19 -: eine Draufsicht auf eine Verstelleinrichtung;
- Figur 20 -: eine Detaildarstellung der Lagerung eines Waagebalkens auf der Spindelmutter;
- Figur 21 -: eine Schnittdarstellung des distalen Endes der Spindellagerung;
- Figur 22 -: eine perspektivische Schrägdraufsicht auf ein Medialglied; sowie
- Figur 23 -: eine Darstellung der mechanischen Komponenten des Prothesenfingers ohne Rahmenteile.

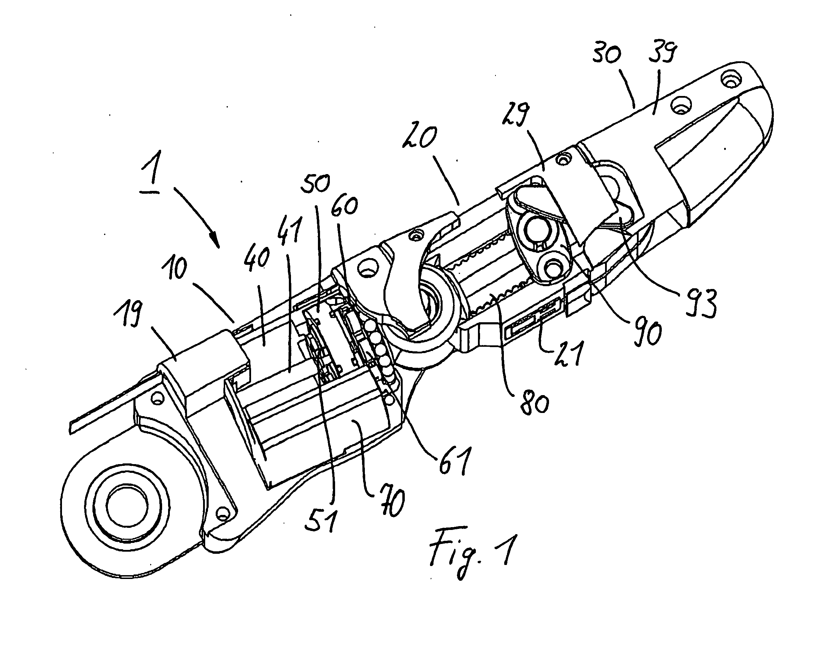

In der Figur 1 ist in einer Teilschnittdarstellung ein Prothesenfinger 1 mit einem Proximalglied 10, einem Medialglied 20 und einem Distalglied 30 dargestellt. Das Proximalglied 10 kann gelenkig angetrieben an einem nicht dargestellten Chassis befestigt sein. Sowohl die Anbindung als auch der Antrieb des Proximalgliedes 10 ist nicht dargestellt.

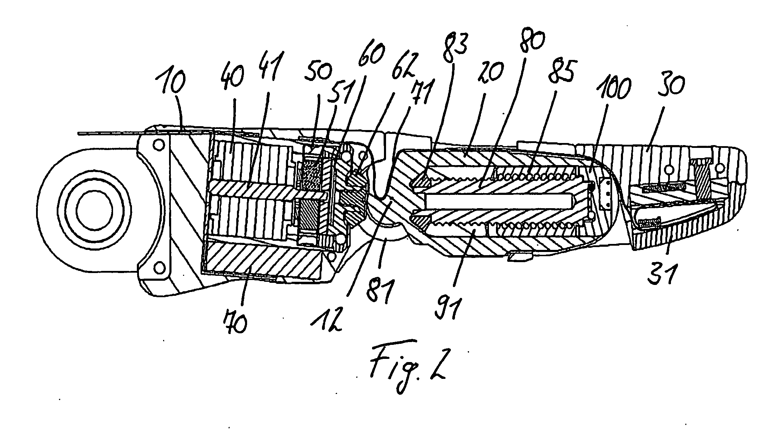

Innerhalb des Proximalgliedes 10 ist ein Motor 40 mit einer Abtriebswelle 41 angeordnet, die in ein Reibradgetriebe 50 mit Reibrädern 51 eingreift. Über das Reibradgetriebe 50 wird ein Gesperre 60 mit Klemmrollen 61 angetrieben, das wiederum mit einem Abtriebskegelrad gekoppelt ist. Das Abtriebskegelrad ist in Figur 2 dargestellt und mit dem Bezugszeichen 62 versehen. Das Abtriebskegelrad 62 greift in ein Kopplungskegelrad 81 ein, das drehbar um eine Drehachse 12 des Medialgliedes 20 um das Proximalglied 10 gelagert ist. Das Kopplungskegelrad 81 treibt ein drehfest auf einer Spindel 80 angeordnetes Antriebskegelrad 83 an. Die Spindel 80 ist auf dem Medialglied 20 gelagert und vorliegend als Kugelumlaufspindel ausgebildet. Auf der Kugelumlaufspindel 80 ist eine Spindelmutter 85 gelagert und wird, je nach Drehrichtung der Spindel 80, in Richtung des distalen oder proximalen Endes der Spindel 80 bewegt. Die Spindelmutter 85 ist somit längsverschieblich und drehfest innerhalb des Medialglieds 20 gelagert. An dem distalen Ende der Spindel 80 ist eine Sperreinrichtung 100 befestigt, die später näher erläutert wird.

An dem distalen Ende des Medialglieds 20 ist ein Distalglied 30 gelenkig angeordnet.

An der Spindelmutter 85 ist ein Waagebalken 90 angeordnet, der in einem im Wesentlichen festen Winkel zu der Längsrichtung der Spindel 80 angeordnet ist. An dem palmaren und dorsalen Ende des Waagebalkens 90 sind Aufnahmen für Hebel 91, 93 ausgebildet, die eine Zug- und Druckübertragung sowie eine Drehung der Hebel 91, 93 innerhalb der Aufnahmen ermöglichen. Die Hebel 91, 93 sind an dem Proximalglied 10 bzw. Distalglied 30 angeordnet. In der vorliegenden Ausgestaltungsform ist der Hebel 91, der das Proximalglied 10 mit dem Waagebalken 90 verbindet, an dem Proximalglied 10 dorsal und an dem Waagebalken 90 palmar angeordnet, während der Hebel 93, der das Distalglied 30 mit dem Waagebalken 90 verbindet, an dem Waagebalken 90 dorsal und an dem Distalglied 30 palmar befestigt ist, wobei sich dorsal und palmar auf die Anordnung relativ zu den Drehachsen der Prothesenfingerglieder bezieht.

In der Figur 1 ist der Prothesenfinger 1 in gestreckter Stellung gezeigt. Die beweglichen Teile sind gliederweise durch Abdeckungen 19, 29, 39 abgedeckt und geschützt, wobei die Gehäuse 19, 29. 39 und die einzelnen Glieder 10, 20, 30 so ausgebildet sind, dass eine entsprechende Verschwenkung der Glieder 10, 20, 30 zueinander ermöglicht wird.

Unterhalb des Motors 40 ist eine Steuereinrichtung 70 bzw. Elektronikeinheit ausgebildet, die über einen flexiblen Leiter 71, der an den Gelenkstellen als Schlaufe ausgebildet ist, um eine Bewegung nachvollziehen zu können, mit Sensoreinrichtungen verbunden ist, die später erläutert werden.

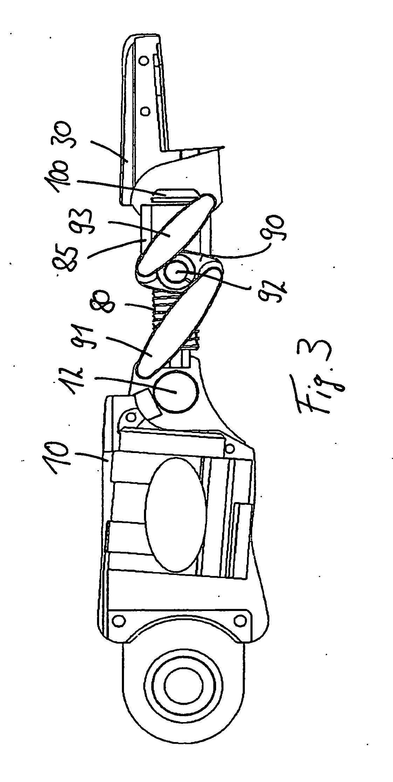

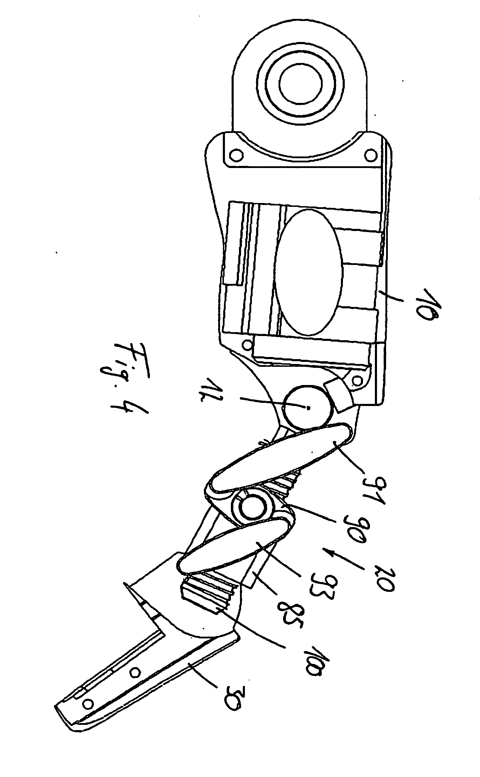

In der Figur 3 ist der Verstellmechanismus innerhalb des Medialglieds 20 in Einzeldarstellung gezeigt. Die Antriebseinrichtungen sowie die Getriebeelemente sind aus Gründen der Übersichtlichkeit freigeschnitten. Die Spindel 80, die statt einer Kugelumlaufspindel auch als eine normale Spindel ausgebildet sein kann, ist drehbar innerhalb des Medialglieds 20 gelagert. Die Lagerstelle am distalen Ende kann durch eine Sperreinrichtung 100, z.B. in Gestalt einer elastischen Kappe, ausgebildet sein, deren Funktion später erläutert werden wird. An dem dorsalen Ende des Waagebalkens 90, der auf einem Zapfen 92 der Spindelmutter 85 angeordnet ist, sind dorsal und palmar die zug- und druckstabilen Hebel 91, 92 befestigt. Der Verbindungshebel 91 zum Proximalglied 10 ist dorsal der Drehachse 12 an dem Proximalglied 10 befestigt, während er am Waagebalken 90 palmar, also unterhalb der Drehachse 12 angeordnet ist. In der gleichen Orientierung ist der Verbindungshebel 93 an dem Distalglied 30 angebracht, wobei die Befestigungsstelle an dem Waagebalken 90 oberhalb der Drehachse des Distalgliedes 30, die aus Übersichtlichkeitsgründen nicht dargestellt ist, angelenkt, am Distalglied 30 selbst jedoch unterhalb angelenkt ist. Wird nun die Kugelumlaufspindel 80 in Uhrzeigerrichtung, gesehen in Distalrichtung, angetrieben, verlagert sich die Spindelmutter 85, die durch die Hebel 91, 93 oder durch eine Ausformung der Innenwandung des Medialgliedes 20 drehfest gelagert ist und in der die Umlaufkugeln umlaufen, in proximale Richtung. In der mittleren Position ist sie in der Figur 4 dargestellt.

Die Figur 4 zeigt den Prothesenfinger 1 in leicht angewinkelter Position, bei der das Medialglied 20, dessen Längserstreckung mit der Längserstreckung der Kugelumlaufspindel 80 übereinstimmt, ungefähr 45 ° zur gestreckten Stellung dorsal verschwenkt ist. Dies ergibt sich aus der Geometrie der Anlenkpunkte der Hebel 91, 93 an dem Waagebalken 90 und an dem Distalglied 30 bzw. Proximalglied 10. Aufgrund der im Wesentlichen drehstarren Orientierung des Waagebalkens 90 an der Spindelmutter 85 wird gleichzeitig mit der Verschwenkung des Medialglieds 20 relativ zu dem Proximalglied 10 das Distalglied 30 relativ zu dem Medialglied 20 verschwenkt. Es liegt also eine Bewegungskopplung des Hebelgestänges der Waagebalken-Hebelkinematik vor, so dass eine harmonische Beugebewegung bei der Beugung des Medialgliedes 20 zusammen mit dem Distalglied 30 durchgeführt wird.

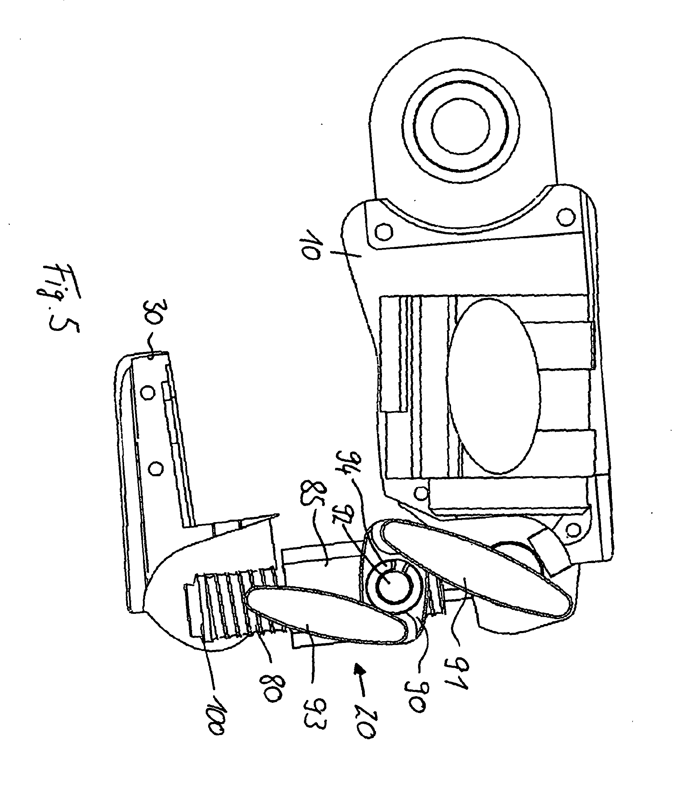

In der Figur 5 ist der Prothesenfinger 1 bei abgewinkeltem Finger dargestellt. Das Distalglied 30 steht nahezu parallel zu dem Proximalglied 10, die Spindelmutter 85 ist nahezu vollständig in Richtung des Proximalgliedes 10 verlagert, so dass sowohl das Medialglied 20 als auch das Distalglied 30 jeweils in einem nahezu rechten Winkel zu dem proximal angeordneten Glied stehen.

Durch die gewählte Anordnung der Hebel 91, 93 am Proximal- und Distalglied 30, 10, der jeweiligen Anlenkpunkte der Hebel 91, 93 an dem Proximal- und Distalglied 10, 30 sowie die Dimensionierung des Waagebalkens 90 wird vermieden, dass die Hebel 91, 93 durch die Drehachsen 12, 23 des Medial- oder Distalgliedes 20, 30 gehen, so dass eine Totlage vermieden wird. Ist eine solche Totlage gewünscht, müssten die Hebel 91, 93 durch die Drehachsen 12, 23 hindurch gehen.

Ist der Waagebalken 90 drehfest an der Spindelmutter 85 angeordnet, so stellt sich ein festes Verhältnis der Verschwenkbewegungen des Medialgliedes 20 und des Distalgliedes 30 ein. Durch die Anordnung der Hebel 91, 93 in einer anderen Art und Weise ist es möglich, ein Voreilen des Medialgliedes 20 zu ermöglichen, so dass eine eher der natürlichen Stellung angenäherte Endposition des Prothesenfingers 1 ermöglicht wird. Eine solche Stellung wäre dann erreicht, wenn das Medialglied 20 in einer Endstellung von 120 ° zu dem Proximalglied 10 und das Distalglied 30 in einer Endstellung von 90 ° zu dem Medialglied 20 ausgerichtet ist.

In den Figuren 3 bis 5 ist zu erkennen, dass über eine Feder 94 der Waagebalken 90 gegenüber einem Anschlag, der später erläutert werden wird, in einer Normalposition gehalten wird. Dadurch ist eine leichte Verdrehbarkeit des Waagebalkens 90, vorliegend entgegen dem Uhrzeigersinn, möglich, so dass bei Kontakt des Medialgliedes 20 mit einem Objekt, so dass sich das Medialglied 20 nicht weiterbewegen kann, der Positionswinkel des Waagebalkens 90 relativ zur Längsbewegungsachse verändert. Dadurch wird die Position des Anlenkpunktes des Hebels 93 durch die Drehung des Waagebalkens 90 verändert, so dass sich das Distalglied 30 über einen beschränkten Bereich, der durch die Größe des Bewegungswinkels des Waagebalkens 90 definiert wird, weiterbewegen kann. Grundsätzlich ist neben der Ausbildung mittels einer Feder 94 und nur einer freien Drehrichtung des Waagebalkens auch eine beidseitig elastische Lagerung, beispielsweise durch ein Elastomerelement oder durch Federn, möglich, so dass eine Nachgiebigkeit und eine Nachführung in beiden Richtungen erfolgen kann.

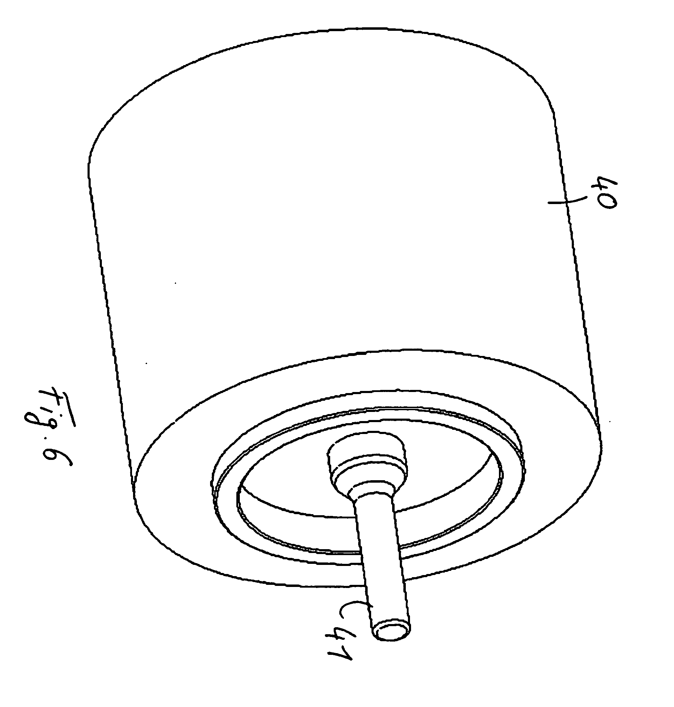

In der Figur 6 ist ein Antrieb eines Elektromotors 40 mit einer Abtriebswelle 41 dargestellt. Die Abtriebswelle 41 ragt im montierten Zustand in ein Reibradgetriebe 50 hinein, das vorliegend drei Reibräder 51 aufweist, die innerhalb eines Laufringes 52 umlaufen. Jenseits des nicht dargestellten Motors 40 ist der Abtrieb 53 gezeigt. An dem äußeren Umfang des Reibradgetriebes 50 sind Vorsprünge 57 angeordnet, mit denen eine drehfeste Lagerung des Laufringes 52 innerhalb des Proximalgliedes realisiert werden kann.

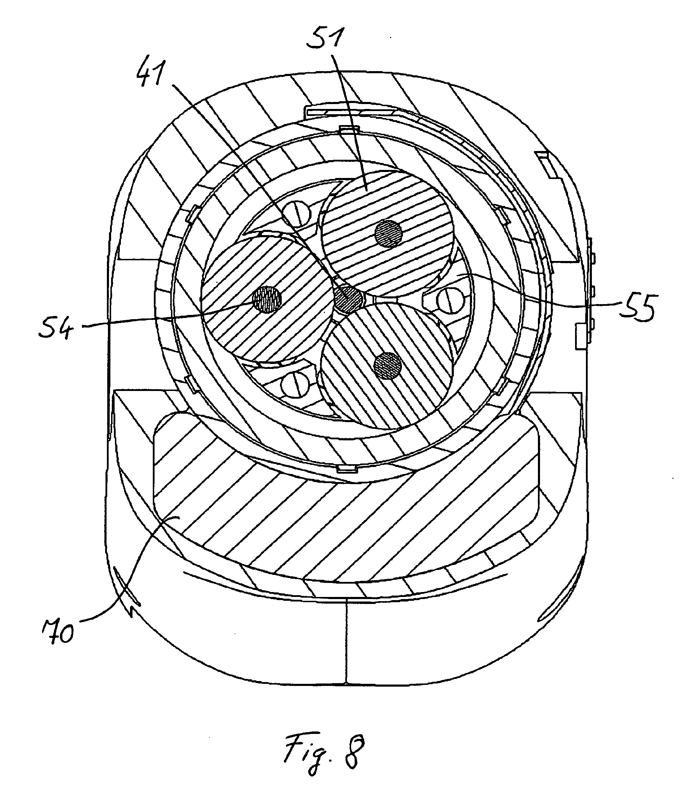

Die Figur 8 zeigt einen Schnitt senkrecht zur Abtriebsachse 41 durch das Reibradgetriebe 50 aus Distalrichtung. Die drei Reibräder 51 liegen an der Abtriebswelle 41 an und lassen somit die auf Achsen 54 gelagerten Reibräder 51 innerhalb des Laufringes 52 umlaufen. Aus dieser Ansicht ist die unterhalb des Motors 40 angeordnete Steuerelektronik 70 gut zu erkennen.

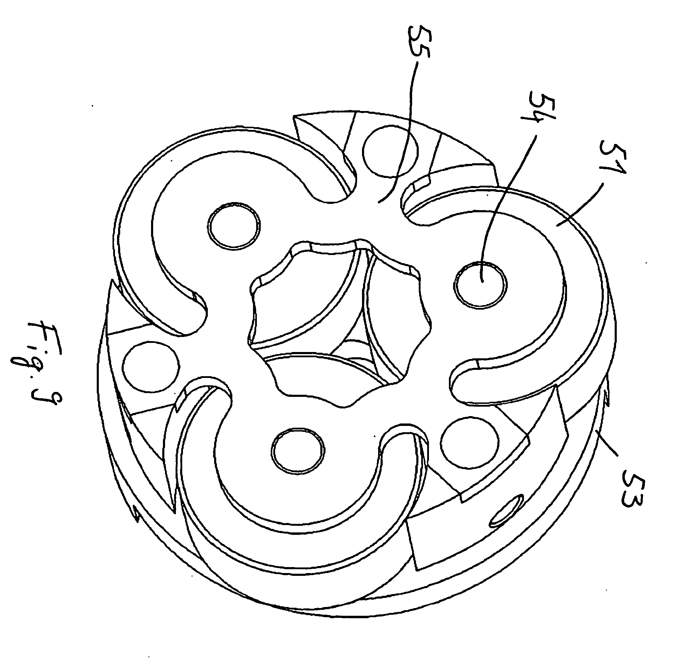

Figur 9 zeigt antriebsseitig das Reibradgetriebe 50 mit den Reibrädern 51, die auf den Achsen 54 eines Reibradträgers 55 gelagert sind, sowie die Abtriebsseite 53.

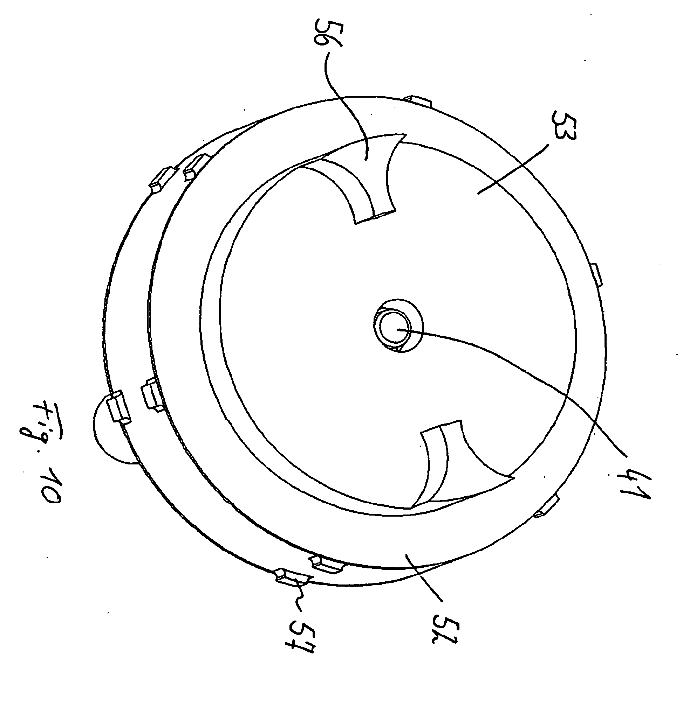

Figur 10 zeigt das Reibradgetriebe 50 abtriebsseitig, wobei der Abtrieb 53 axial vorstehende Ausrückelemente 56 aufweist, die gleichzeitig zum Antrieb eines Gesperres 60 dienen, das schon in den Figuren 1 und 2 in Gesamtdarstellung gezeigt ist.

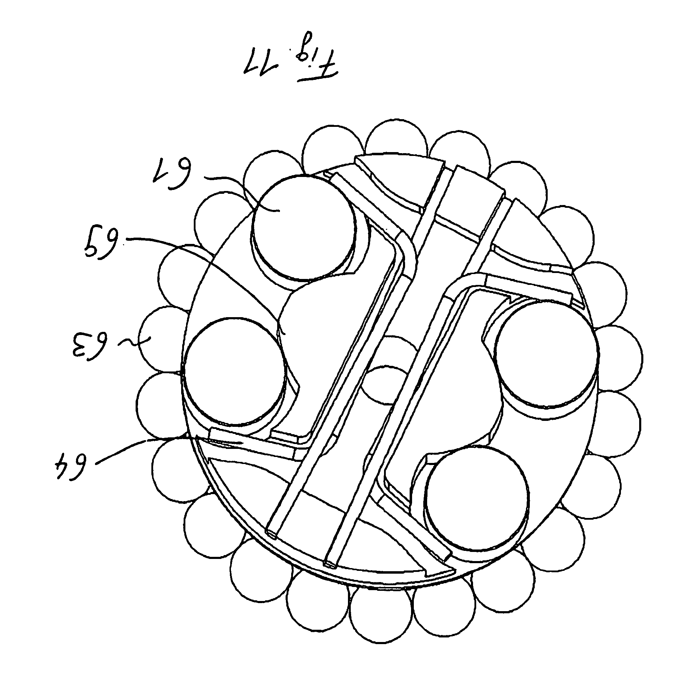

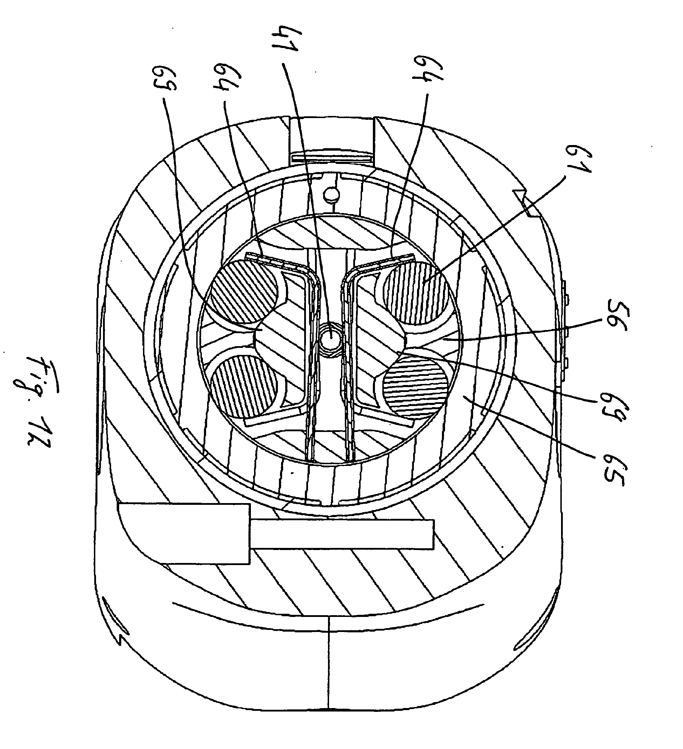

Die Figur 11 zeigt das Gesperre 60 mit vier Klemmrollen 61, die sich, wie in Figur 12 dargestellt, gegenüber einem Klemmring 65 abstützen. Über Federn 64 werden die Klemmrollen 61 gegen Anlageschultern 69 auf einem Träger 68 gedrückt, so dass sie ohne Beaufschlagung mit einem Antriebsmoment stets in reibschlüssigem Kontakt mit dem Klemmring 65 stehen. Wird nun das Abtriebselement 53 mit den Ausrückelementen 56 im Uhrzeigersinn verdreht, werden die jeweils gegenüberliegenden Klemmrollen 61 gegen die Federn 64 gedrückt und außer Eingriff mit dem Klemmring 65 gebracht, so dass das Gesperre 60 gedreht werden kann. Die nicht in Kontakt mit dem Ausrückelement 56 tretenden Klemmrollen 61 werden gegen die entsprechenden Enden der Federn 64 gedrückt und laufen aufgrund der geometrischen Abmessungen der Schultern 69 mit. Wird der Motor 40 und damit auch der Abtrieb 53 des Reibradgetriebes 50 in die andere Richtung gedreht, werden die Ausrückelemente 56 die jeweils schräg gegenüberliegenden Klemmrollen 61 außer Eingriff bringen.

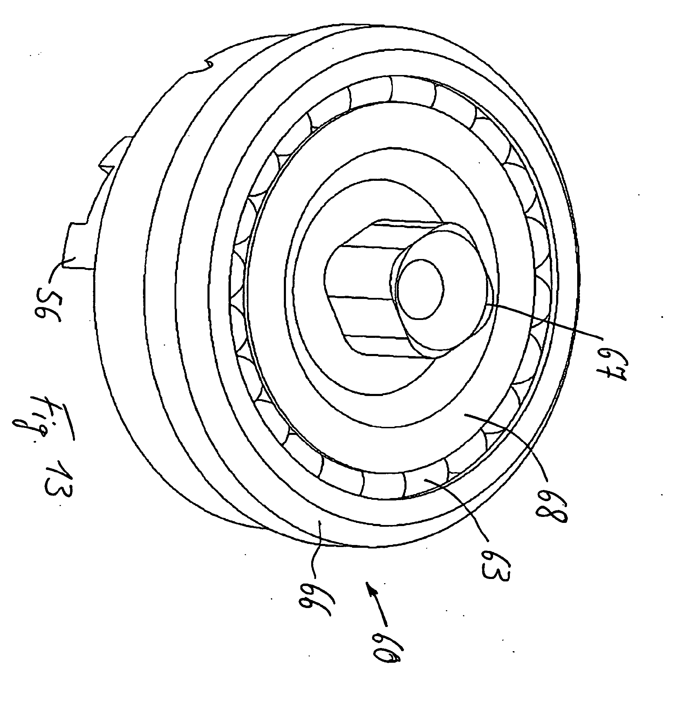

In der Figur 13 ist in einer perspektivischen Gesamtansicht das Gesperre 60 gezeigt, das über Kugeln 63 an dem äußeren Lagerring 66 gelagert ist. An dem Träger 68 ist abtriebsseitig ein Abtriebszapfen 67 angeordnet, auf den ein Abtriebszahnrad 62 aufgesteckt werden kann.

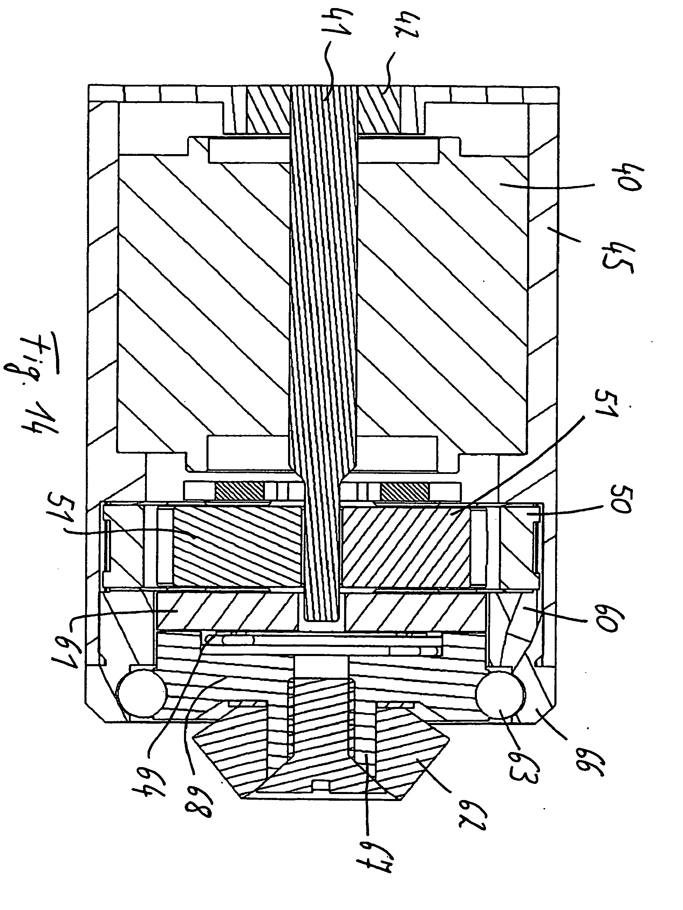

In der Figur 14 ist eine Schnittdarstellung in die Baueinheit, bestehend aus Motor 40, Reibradgetriebe 50 und Gesperre 60 dargestellt, die in einem gemeinsamen Gehäuse 45 untergebracht sind. Auf den Abtriebszapfen 67 ist ein Abtriebskegelrad 62 aufgesteckt oder daran festgelegt. Wird nun abtriebsseitig über das Abtriebszahnrad 62 ein Moment eingeleitet, verkeilen sich die Klemmrollen 61 mit dem Klemmring 65, so dass das Reibradgetriebe 50 und der Motor sich nicht bewegen können. Diese Fixierung ist notwendig, da die Spindel 80, die als Kugelumlaufspindel ausgebildet ist, einen sehr hohen Wirkungsgrad ohne Selbsthemmungsneigung aufweist. Die Außenlagerung des Gesperres 60 über die Kugeln 63 im Lagerring 66 dient zur zusätzlichen Fixierung und Ausrichtung des Abtriebskegelrades 62. In der Figur 14 ist auch zu sehen, dass die Abtriebswelle 41 des Motors 40 proximal an dem Gehäuse 45 des Motors 40 in einem Lager 42 gelagert ist, während die distale Lagerung innerhalb des Reibradgetriebes 50 erfolgt. Dadurch kann axialer Bauraum eingespart werden, da keine zweite Lagerung der Abtriebswelle 41 in dem Motor 40 selbst vorgesehen sein muss.

Die Einheit aus Motor 40, Reibradgetriebe 50 und Gesperre 60 kann auch separat gefertigt und in anderen Antrieben eingesetzt werden, so dass hierin eine eigenständige Lösung zu erkennen ist.

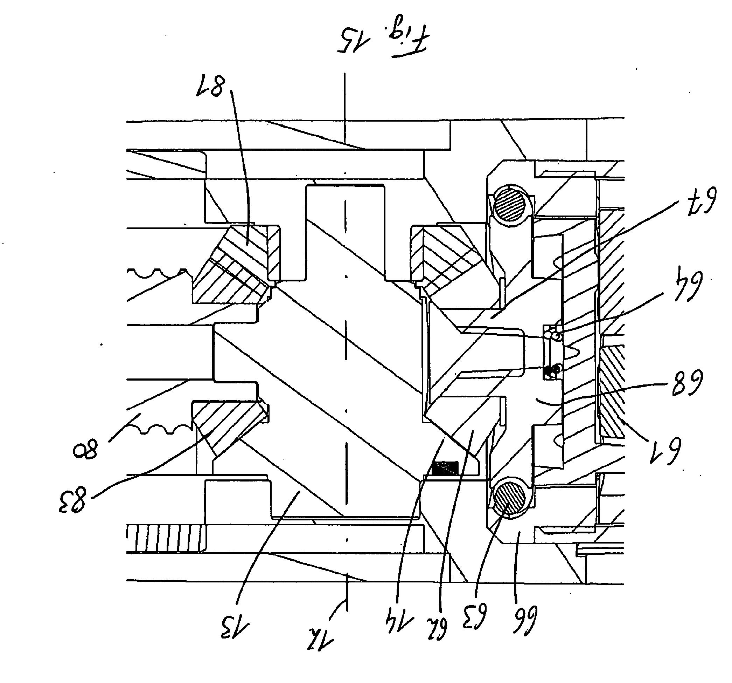

In der Figur 15 ist in Schnittdarstellung die Getriebeanordnung und die Kraftübertragung von dem Gesperre 60 über das Abtriebskegelrad 62 auf die Kugelumlaufspindel 80 dargestellt. Um die Drehachse 12 des Medialgliedes 20 ist ein Kopplungskegelzahnrad 81 gelagert. Dieses ist um einen Zapfen 13 herum drehbar angeordnet. Der Zapfen 13 weist eine Stützfläche 14 für das Abtriebskegelrad 62 auf, diese tritt jedoch mit diesem nicht in Eingriff. Auf der der Stützfläche 14 gegenüberliegenden Seite ist das drehbare Kopplungskegelrad 81 drehbar gelagert und greift in das Antriebskegelrad 83 ein, das drehfest mit der Spindel 80 verbunden ist. Um eine gewisse axiale Beweglichkeit zu erreichen, kann das Kegelrad 83 drehfest und längsverschieblich an der Spindel 80 gelagert sein. Durch die Anordnung des Kopplungszahnrades 81 um die Drehachse 12 kann unabhängig von der Winkelstellung des Medialgliedes 20 eine gleichmäßige Kraftübertragung erfolgen.

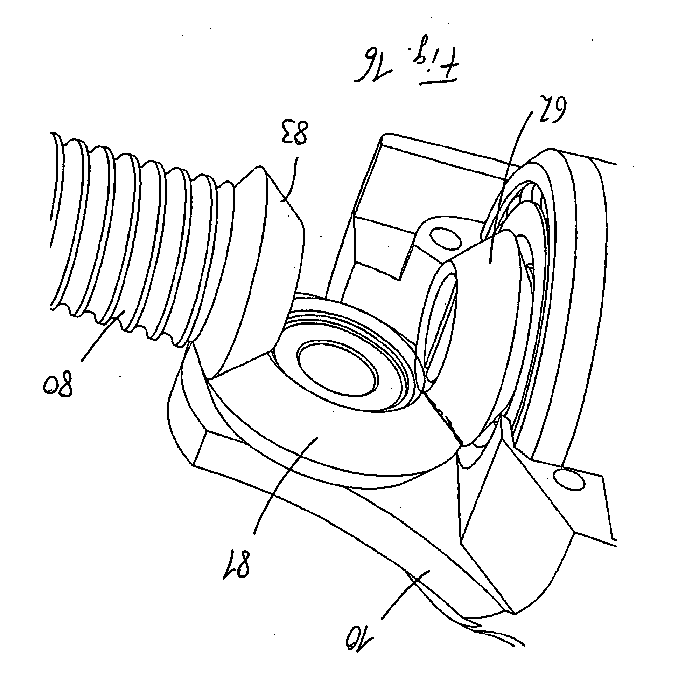

In der Figur 16 ist eine perspektivische Teilschnittdarstellung mit dem Abtriebskegelzahnrad 62, dem Kopplungskegelzahnrad 81 und dem Antriebskegelrad 83 gezeigt.

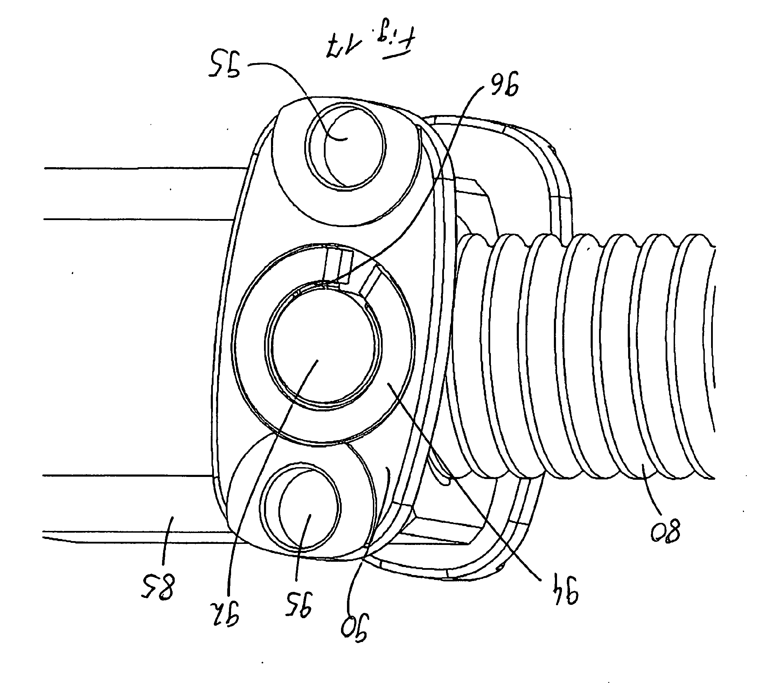

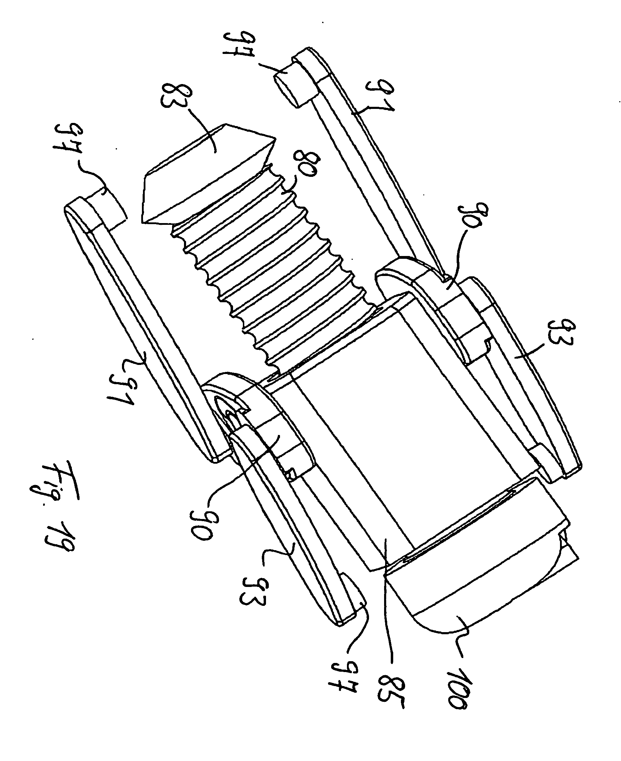

In der Figur 17 ist in einer Detaildarstellung die Spindelmutter 85 an der Spindel 80 gezeigt. An der Spindelmutter 85 ist der Waagebalken 90 an einem Zapfen 92 gelagert. An dem Zapfen 92 ist eine Nase 96 angeordnet, über die sich die Feder 94 abstützt, so dass sich der Waagebalken 90 über die Federvorspannung in der dargestellten Normalposition befindet. Aufgrund des Freiraumes zwischen den Federenden kann eine begrenzte Verschwenkbewegung entgegen dem Uhrzeigersinn erfolgen. Zur Synchronisierung der Bewegungen und zur Vergleichmäßigung der Ausrichtung der beidseitig neben der Spindel 80 an der Spindelmutter 85 angeordneten Waagebalken 90 können diese auch über ein Kopplungselement miteinander verbunden sein, so dass eine Brücke oder ein Rahmen ausgebildet wird. An den äußeren Enden des Waagebalkens 90 sind runde Ausnehmungen 95 zur Aufnahme von Zapfen 97 der Hebel 91, 93, wie in Figur 19 dargestellt, vorgesehen.

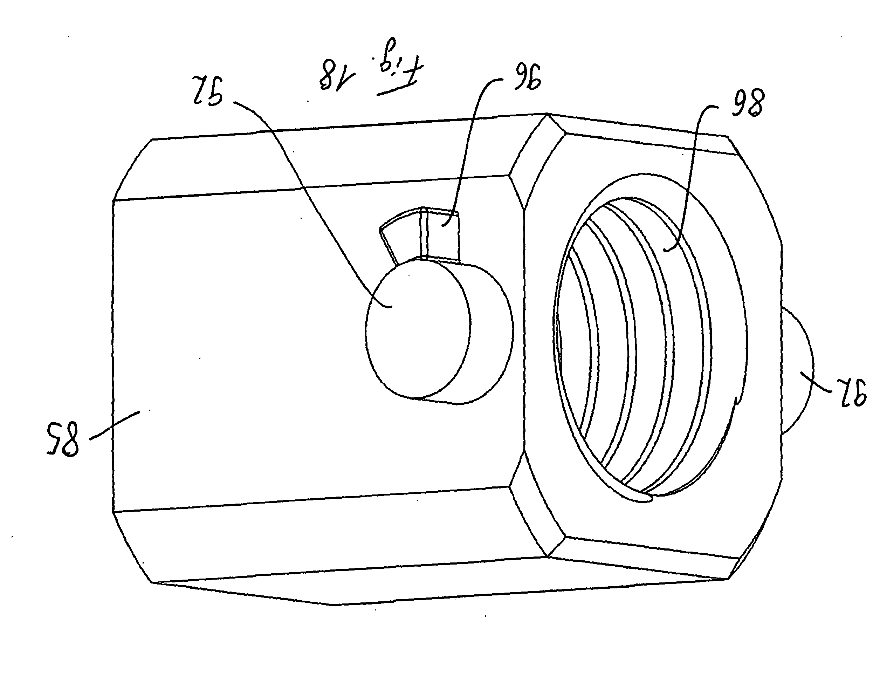

Figur 18 zeigt eine Einzeldarstellung der Spindelmutter 85, die als Kugelumlaufspindelmutter ausgebildet ist. Kugelbahnen 86 sind in der Bohrung der Spindelmutter 85 ausgebildet. Die Zapfen 92 ragen beidseitig senkrecht zur Verlagerungsrichtung der Spindelmutter 85 ab und weisen die Nasen 96 auf.

In der Figur 19 ist die Waagebalken-Hebelkinematik in einer Draufsicht dargestellt. Deutlich sind die beidseitig an der Spindelmutter 85 angeordneten Waagebalken 90 sowie die symmetrisch angeordneten Hebel 91, 93 zur Verbindung der Waagebalken 90 mit dem angrenzenden Distal- und Proximalglied 30, 10 zu erkennen. An dem distalen Ende der Spindel 80 ist die Lagerung und Sperreinrichtung 100 gezeigt, am proximalen Ende das Antriebskegelrad 83.

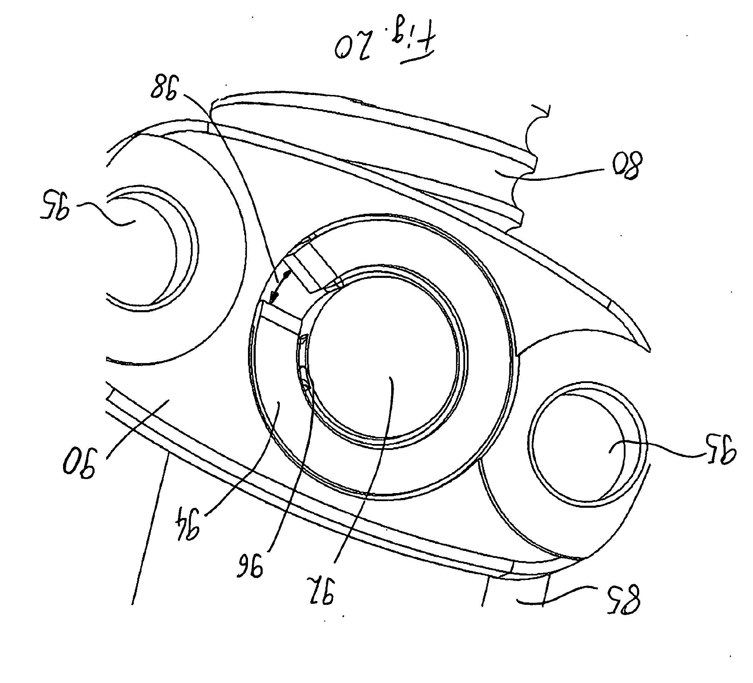

In der Figur 20 ist im Detail die bewegliche Lagerung des Waagebalkens 90 auf der Spindelmutter 85 dargestellt. Um den Zapfen 92 mit der Nase 96 ist der Waagebalken 90 angeordnet. Ein Schwenkbereich 98 wird freigelassen und ermöglicht so eine Drehung des Waagebalkens 90 entgegen der Uhrzeigerrichtung. Die Feder 94 dient dazu, den Waagebalken 90 während der Beugung des Prothesenfingers 1 in dem richtigen Winkel relativ zu der Längserstreckung der Spindel 80 zu halten. Bei einem nicht gleichmäßigen Kontaktieren des Medial- und Distalgliedes 20, 30 mit einem Gegenstand kann der Winkel verändert werden, so dass eine Differenzialwirkung und eine leichte Nachführung des später in Kontakt tretenden Gliedes erfolgen kann. Anders als dargestellt, kann die Ringfeder 94 auch eine Druckfeder sein, beispielsweise als einem elastischen Bauteil oder einem Kunststoff bestehen. Ebenfalls ist eine beidseitig verschwenkbare Lagerung des Waagebalkens 90 möglich.

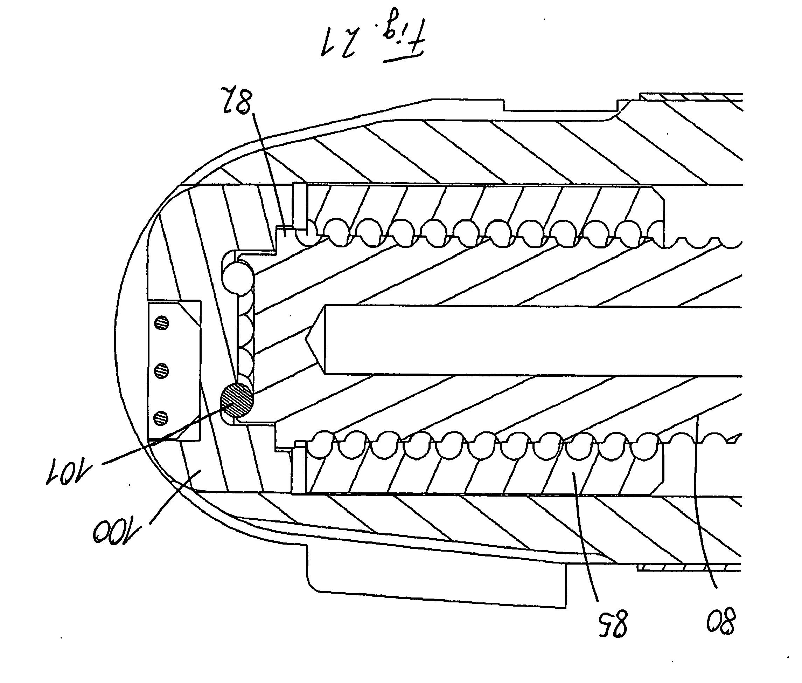

In der Figur 21 ist eine Überlastsicherung dargestellt, die dazu bestimmt ist, sehr hohe auftretende Kräfte, die gegen das Distalglied 30 in Fingerabwicklungsrichtung auftreten, nicht über das Waagebalken-Hebelsystem, sondern über die Kugelumlaufspindel 80 und das Medialgliedgehäuse 29 abzuleiten. Dadurch wird bei unvorhergesehenen, hohen Lasten, sogenannten Schocklasten, das Kinematiksystem vor einer Zerstörung geschützt. Bei kleinen Lasten in Fingerabwicklungsrichtung berührt die Kugelumlaufspindel 80 die Sperreinrichtung 100 nicht, vielmehr ist zwischen einem an der Spindel 80 ausgebildeten Absatz 82 und einer korrespondierenden Reibfläche 180 der Sperreinrichtung 100 ein kleiner Freiraum gelassen. Die Kugeln 101 ermöglichen eine leichte Drehung im Normalbetrieb des Antriebes und bilden die distale Lagerung der Spindel 80. Bei einer Krafteinleitung über das Distalglied 30 wird die Kugelspindelmutter 85 längsverschoben, wodurch aufgrund der fehlenden Selbsthemmung des Kugelumlaufspindelgetriebes eine Axialverlagerung der Spindel 80 erfolgt. Bei größeren Lasten wird somit der Spalt zwischen der Reibfläche 180 und dem Absatz 82 aufgrund der elastischen Ausgestaltung der Sperreinrichtung, beispielsweise als Kunststoffkappe, überwunden, so dass die Spindel 80 abgebremst wird. Alternativ zu einem reibschlüssigen Kontakt kann dieser formschlüssig ausgebildet sein.

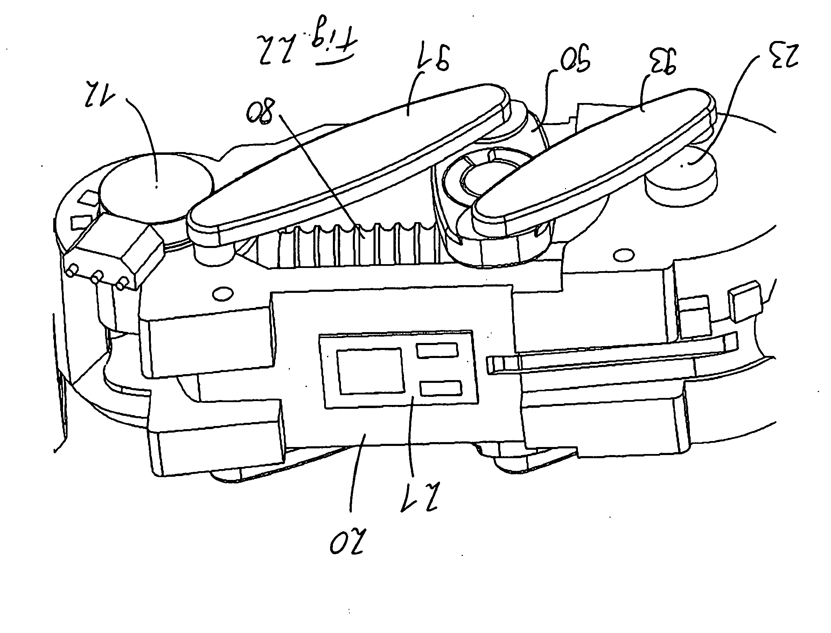

In der Figur 22 ist in einer Draufsicht das Medialglied 20 mit einem auf der Oberseite angeordneten Dehnmessstreifen 21 gezeigt. Solche Sensoren 21 sind notwendig, um die inneren strukturellen Spannungen, beispielsweise als Folge durch die Belastung, zu messen, um eine Rückmeldung an die Elektronikeinheit 70 zu gewährleisten. Ebenfalls ist es vorgesehen, dass weitere Sensoren innerhalb des Prothesenfingers 1 angeordnet sind, die über die relative Position der Glieder 10, 20. 30 zueinander und gegebenenfalls zu einem Chassis Daten liefern. Diese Daten werden über den Leiter 71 zu der Steuereinheit 70 geleitet. Dort werden sie ausgewertet und können dann über einen Bus einer zentralen Steuereinheit übermittelt werden. Dadurch verringert sich die Anzahl der aus den Prothesenfingern 1 herauszuführenden Leitungen signifikant.

Ebenfalls ist ein taktiler Sensor 31 an der palmaren und distalen Seite des Distalgliedes 30 vorgesehen, um eine haptische Rückmeldung anbieten zu können. Ebenfalls können dadurch Griffkräfte gesteuert werden.

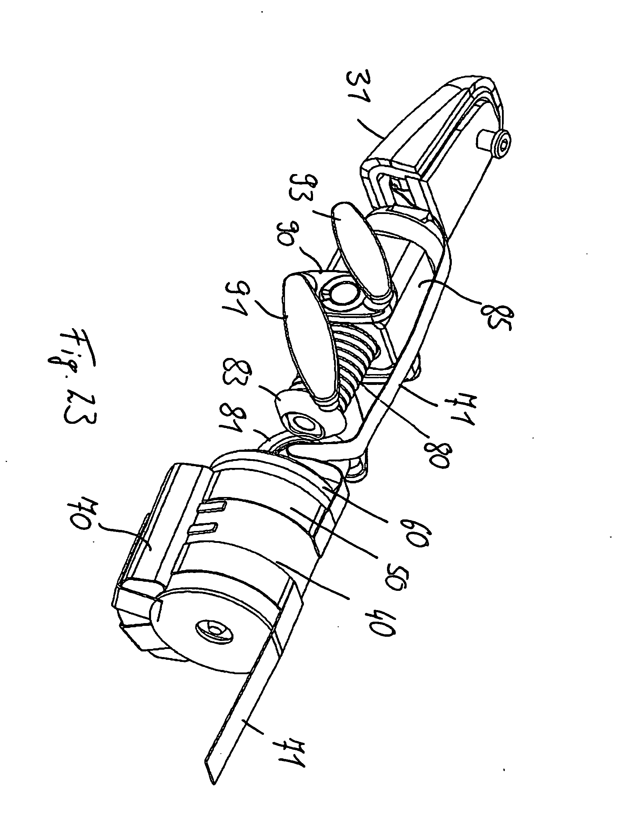

In der Figur 23 ist der Aufbau des Prothesenfingers ohne Gehäuse der einzelnen Fingerglieder 10, 20, 30 dargestellt. Die Verkabelung über den flexiblen Leiter 71 erfolgt dergestalt, dass keine Knickungen während des Betriebes vorhanden sind.

Die Messung des Winkels kann über einen Halleffektsensor erfolgen, der in allen Gelenken angeordnet ist.

Sämtliche Gehäuse und Träger der einzelnen Glieder können zweiteilig ausgebildet sein, um die Montierbarkeit an den jeweiligen Anschlussgliedern zu ermöglichen.

## Patentansprüche

1. Prothesenfinger (1) mit einem Proximalglied (10), einem Medialglied (20) und einem Distalglied (30), die verschwenkbar aneinander gelagert sind, und einem in dem Prothesenfinger (1) angeordneten Motor (40), der über eine Getriebeeinrichtung das Medialglied (20) relativ zu dem Proximalglied (10) verdreht, **dadurch gekennzeichnet, dass** in dem Medialglied (20) ein längsbeweglicher Waagebalken (90) angeordnet ist, der über Hebel (91, 93) mit dem Proximalglied (10) und dem Distalglied (30) verbunden ist.

2. Prothesenfinger nach Anspruch 1, **dadurch gekennzeichnet, dass** der Motor (40) mit einer Spindel (80) gekoppelt ist, die drehbeweglich in dem Medialglied (20) gelagert ist und auf der eine Spindelmutter (85) längsverschieblich und drehfest angeordnet ist.

3. Prothesenfinger nach Anspruch 2, **dadurch gekennzeichnet, dass** die Spindel (80) als eine Kugelumlaufspindel ausgebildet ist.

4. Prothesenfinger nach einem der voranstehenden Ansprüche, **dadurch gekennzeichnet, dass** die Hebel (91, 93) beidseitig neben der Spindelmutter (85) angeordnet sind.

5. Prothesenfinger nach einem der voranstehenden Ansprüche, **dadurch gekennzeichnet, dass** die Spindel (80) und der Motor (40) über ein Kegelradgetriebe (62, 81, 83) miteinander gekoppelt sind.

6. Prothesenfinger nach einem der voranstehenden Ansprüche, **dadurch gekennzeichnet, dass** zwei Waagebalken (90) beiderseits der Bewegungsachse der Waagebalken (90) angeordnet sind.

7. Prothesenfinger nach einem der voranstehenden Ansprüche, **dadurch gekennzeichnet, dass** der Waagebalken (90) unter Federvorspannung in einer Normalposition gehalten ist.

8. Prothesenfinger nach einem der Ansprüche 2 bis 7, **dadurch gekennzeichnet, dass** die Spindel (80) axial verlagerbar gelagert ist und mit einer Sperreinrichtung (100) versehen ist, die bei Überschreiten eines festgelegten Verschiebeweges mit der Spindel (80) in Eingriff tritt.

9. Prothesenfinger nach Anspruch 8, **dadurch gekennzeichnet, dass** die Sperreinrichtung (100) als Formschluss- oder Reibschlussbremse ausgebildet ist.

10. Prothesenfinger nach Anspruch 8 oder 9, **dadurch gekennzeichnet, dass** die Sperreinrichtung (100) als eine elastische Aufnahme mit Reibflächen (180) oder Formschlusselementen ausgebildet ist.

11. Prothesenfinger nach einem der voranstehenden Ansprüche, **dadurch gekennzeichnet, dass** an jedem Gelenk des Prothesenfingers (1) Sensoreinrichtungen zur Erfassung der Winkelstellung und/oder zur Erfassung der Belastung der Bauteile, insbesondere Dehnmessstreifen, vorgesehen sind,

12. Prothesenfinger nach einem der voranstehenden Ansprüche, **dadurch gekennzeichnet, dass** eine Steuerelektronikeinheit (70) des Motors (40) und Auswerteeinheit im Prothesenfinger (1) angeordnet ist.

13. Prothesenfinger nach einem der voranstehenden Ansprüche, **dadurch gekennzeichnet, dass** der Motor (40) mit einem Untersetzungsgetriebe (50) gekoppelt ist, das insbesondere als ein ein- oder mehrstufiges Planetengetriebe, insbesondere als ein Zahnrad- oder Reibradgetriebe ausgebildet ist.

14. Prothesenfinger nach einem der voranstehenden Ansprüche, **dadurch gekennzeichnet, dass** dem Motor (40) ein Gesperre (60) nachgeordnet ist, das eine abtriebsseitige Lasteinleitung sperrt und eine antriebsseitige Drehung weiterleitet, wobei das Gesperre (60) insbesondere als ein in beide Drehrichtungen wirkender Klemmrollenfreilauf ausgebildet ist.

## Claims

1. A prosthetic finger (1) with a proximal member (10), a medial member (20) and a distal member (30) that are mounted pivotably on one another, and with a motor (40) which is arranged in the prosthetic finger (1) and which, via a gear mechanism, rotates the medial member (20) relative to the proximal member (10), **characterized in that** the medial member (20) accommodates a longitudinally movable balance arm (90) which is connected via levers (91, 93) to the proximal member (10) and to the distal member (30).

2. The prosthetic finger as claimed in claim 1, **characterized in that** the motor (40) is coupled to a spindle (80), which is mounted movably in rotation in the medial member (20) and on which a spindle nut (85) is arranged in a longitudinally displaceable and rotationally fixed manner.

3. The prosthetic finger as claimed in claim 2, **characterized in that** the spindle (80) is designed as a recirculating ball spindle.

4. The prosthetic finger as claimed in one of the preceding claims, **characterized in that** the levers (91, 93) are arranged on both sides next to the spindle nut (85).

5. The prosthetic finger as claimed in one of the preceding claims, **characterized in that** the spindle (80) and the motor (40) are coupled to each other via a bevel gear (62, 81, 83).

6. The prosthetic finger as claimed in one of the preceding claims, **characterized in that** two balance arms (90) are arranged on both sides of the axis of movement of the balance arms (90).

7. The prosthetic finger as claimed in one of the preceding claims, **characterized in that** the balance arm (90) is held in a normal position by spring pretensioning.

8. The prosthetic finger as claimed in one of claims 2 through 7, **characterized in that** the spindle (80) is mounted so as to be axially movable and is provided with a blocking mechanism (100) which engages with the spindle (80) when a fixed displacement path is exceeded.

9. The prosthetic finger as claimed in claim 8, **characterized in that** the blocking mechanism (100) is designed as a form-fit brake or friction brake.

10. The prosthetic finger as claimed in claim 8 or 9, **characterized in that** the blocking mechanism (100) is designed as an elastic recess with friction surfaces (180) of form-fit elements.

11. The prosthetic finger as claimed in one of the preceding claims, **characterized in that** sensor devices are arranged on each joint of the prosthetic finger (1) in order to detect the angular position and/or for detecting the loading of the structural parts, in particular strain gauges.

12. The prosthetic finger as claimed in one of the preceding claims, **characterized in that** a control electronics unit (70) of the motor (40) and evaluation unit are arranged in the prosthetic finger (1).

13. The prosthetic finger as claimed in one of the preceding claims, **characterized in that** the motor (40) is coupled to a step-down gear (50) and in particular that the step-down gear (50) is designed as a one-stage or multi-stage planetary gear, in particular as a toothed gear or friction gear.

14. The prosthetic finger as claimed in one of the preceding claims, **characterized in that** the motor (40) is followed by a detent (60) that blocks the introduction of a load on the output side and conveys a rotation on the drive side and that the detent (60) is designed in particular as a clamping roller freewheel that acts in both directions of rotation.

## Revendications

1. Doigt de prothèse (1) comprenant un élément proximal (10), un élément médian (20) et un élément distal (30) qui sont montés à pivot l'un à l'autre, et un moteur (40) qui est agencé dans le doigt de prothèse (1) et fait tourner l'élément médian (20) relativement à l'élément proximal (10) par l'intermédiaire d'un dispositif d'entraînement, **caractérisé en ce que** dans l'élément médian (20) est agencé un balancier (90) mobile longitudinalement qui est relié à l'élément proximal (10) et l'élément distal (30) par l'intermédiaire de leviers (91, 93).

2. Doigt de prothèse selon la revendication 1, **caractérisé en ce que** le moteur (4) est couplé à une broche (80) qui est montée mobile en rotation dans l'élément médian (20) et sur laquelle est agencé un écrou de broche (85) déplaçable longitudinalement et fixe en rotation.

3. Doigt de prothèse selon la revendication 2, **caractérisé en ce que** la broche (80) est formé par une vis à recirculation de billes.

4. Doigt de prothèse selon l'une quelconque des revendications précédentes, **caractérisé en ce que** les leviers (91, 93) sont agencés de part et d'autre à côté de l'écrou de broche (85).

5. Doigt de prothèse selon l'une quelconque des revendications précédentes, **caractérisé en ce que** la broche (80) et le moteur (40) sont couplés ensemble par l'intermédiaire d'une transmission à pignons coniques (62, 81, 83).

6. Doigt de prothèse selon l'une quelconque des revendications précédentes, **caractérisé en ce que** deux balanciers (90) sont agencés de part et d'autre de l'axe de mouvement des balanciers (90).

7. Doigt de prothèse selon l'une quelconque des revendications précédentes, **caractérisé en ce que** le balancier (90) est tenu dans une position normale sous précontrainte par ressort.

8. Doigt de prothèse selon l'une quelconque des revendications 2 à 7, **caractérisé en ce que** la broche (80) est montée déplaçable axialement et munie d'un dispositif d'arrêt (100) qui entre en prise avec la broche (80) lorsqu'une course de déplacement préfixée est dépassée.

9. Doigt de prothèse selon la revendication 8, **caractérisé en ce que** le dispositif d'arrêt (100) est formé par un frein par liaison par forme conjuguée ou à friction.

10. Doigt de prothèse selon la revendication 8 ou 9, **caractérisé en ce que** le dispositif d'arrêt (100) est formé par un logement élastique présentant des faces de friction (180) ou des éléments de liaison par forme conjuguée.

11. Doigt de prothèse selon l'une quelconque des revendications précédentes, **caractérisé en ce qu'**à chaque articulation du doigt de prothèse (1) sont prévus des dispositifs capteurs pour détecter la position angulaire et/ou pour détecter la charge des composants, en particulier des jauges de contrainte.

12. Doigt de prothèse selon l'une quelconque des revendications précédentes, **caractérisé en ce qu'**une unité électronique de pilotage (70) du moteur (40) et unité d'exploitation est agencée dans le doigt de prothèse (1).

13. Doigt de prothèse selon l'une quelconque des revendications précédentes, **caractérisé en ce que** le moteur (40) est couplé à un réducteur de vitesse (50), qui est en particulier formé par une transmission planétaire à un ou à plusieurs étages, en particulier une transmission à roues dentées ou à roues de friction.

14. Doigt de prothèse selon l'une quelconque des revendications précédentes, **caractérisé en ce qu'**en aval du moteur (40) est monté un dispositif de blocage (60) qui bloque une charge appliquée du côté mené et transmet une rotation appliquée du côté appliquée du menant, le dispositif de blocage (60) étant formé en particulier par une roue libre à rouleaux de coincement opérant dans les deux sens de rotation.
